# EUROPEAN PATENT SPECIFICATION

(11) **EP 0 369 874 B1**
(45) Date of publication and mention of the grant of the patent: **22.02.1995**
(21) Application number: 89403125.1
(22) Date of filing: 14.11.1989
(51) Int. Cl.: C07D 311/72, A61K 31/355

(54) **Cardioprotective tocopherol analogs**
Herzschützende tocopherolähnliche Verbindungen
Analogues de tocophérol cardioprotectifs

(30) Priority: 14.11.1988 FR 88402854; 14.11.1988 FR 88402853
(43) Date of publication of application: 23.05.1990
(73) Proprietor: MERRELL DOW PHARMACEUTICALS INC., Cincinnati Ohio 45215-6300 (US)
(72) Inventor: Grisar, J. Martin, F-67160 Wissembourg (FR); Petty, Margaret, F-67000 Strasbourg (FR); Bolkenius, Frank, D-7640 Kehl (DE)
(74) Representative: Gillard, Marie-Louise

(56) References cited:
- US-A- 4 321 270

## Description

This invention relates to alkylamino alkylene derivatives of certain 2H-1-benzopyrans, to the intermediates and processes useful for their preparation, to their free-radical scavenger and cellular protective properties and to their end-use application as therapeutic agents.

More specifically this invention relates to alkylamino alkylene derivatives of the formula 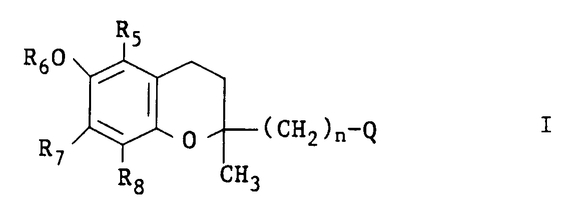
the (R) and (S) enantiomers and racemic mixtures thereof, and the pharmaceutically acceptable salts thereof wherein
- Q: is N^{⊕}R₁R₂R₃·X^{⊖},
- X: is a halide or OS(O)₂R₄, with R₄ being H, C₁₋₆ alkyl, aryl or aralkyl,
- R₁, R₂ and R₃: each individually are a C₁₋₆ lower alkyl,
- R₅: is H or C₁₋₆ alkyl,
- R₆: is H or -C(O)R, R being H or C₁₋₉ alkyl,
- R₇: is H or C₁₋₆ alkyl,
- R₈: is H or C₁₋₆ alkyl and n is an integer of 1 to 6.

As used herein, the moiety (CH₂)ₙ of Formula I wherein n is an integer of one to six represents a C₁₋₆ straight or branched-chain alkylene including such preferred species as methylene, ethylene, propylene, t-butylene, n-butylene, n-hexylene and isopropylene. The term "C₁₋₆ alkyl" includes the straight and branched-chain radicals having up to six carbon atoms with methyl, ethyl, propyl, n-butyl, t-butyl, pentyl and hexyl being representative. The term "-C(O)R" includes those acyl moieties wherein R is H and C₁₋₉ alkyl embracing formyl and the straight and branched-chain alkylcarbonyl moieties having up to ten carbon atoms including methylcarbonyl, ethylcarbonyl, propylcarbonyl, t-butylcarbonyl and n-hexylcarbonyl as preferred representatives. When used, aryl preferably is phenyl or alkylated phenyl, and aralkyl preferably is benzyl or phenethyl.

The moiety "Q" includes those tertiary and quaternary ammonium derivatives wherein NR₁R₂ represents a dialkylamine attached to the alkylene moiety and N^{⊕}R₁R₂R₃·X^{⊖} represents a quaternary ammonium derivative. In each instance the R₁, R₂ and R₃ moieties represent a C₁₋₆ alkyl radical. Although it is preferred to have the R₁, R₂ and R₃ alkyl radicals the same, this invention includes those amines wherein the alkyl radicals are different. Preferably these radicals are methyl and ethyl. Of course, in those instances wherein Q represents a quaternary amine, such compounds would not be utilized as acid addition salts, whereas the tertiary amines preferentially are utilized in the form of their acid addition salts, preferably the hydrochloride or hydrobromide.

The term "pharmaceutically acceptable acid addition salts" embraces those salts capable of being formed by the interaction of an organic or inorganic acid with a pharmaceutical base compound to yield a non-toxic pharmaceutically acceptable entity.
. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono-, di- and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Either the mono- or the di-acid salts can be formed, and such salts can exist in either a hydrated or a substantially anhydrous form. In general, salts of these compounds are crystalline materials which are soluble in water and various hydrophilic organic solvents.

In general the compounds of Formula 1 may be prepared by standard chemical processes and techniques analogously known in the art. In practice, the preparation of the compounds of Formula I conveniently utilizes 3,4-dihydro-2,5,7,8-tetramethyl-2H-1-benzopyran-2-ols as starting materials which, for the most part, are known compounds. In those instances wherein any specific starting material is not known then such compounds may readily be prepared using the standard procedures analogously known in the art as well as by applying such processes as would be reasonably expected to produce the desired starting materials.

The preparation of the 3,4-dihydro-2,5,7,8-tetramethyl-2H-1-benzopyran-2-ols and their conversion to the final products of Formula I is depicted in the following reaction schemes.

### Preparation of Intermediates

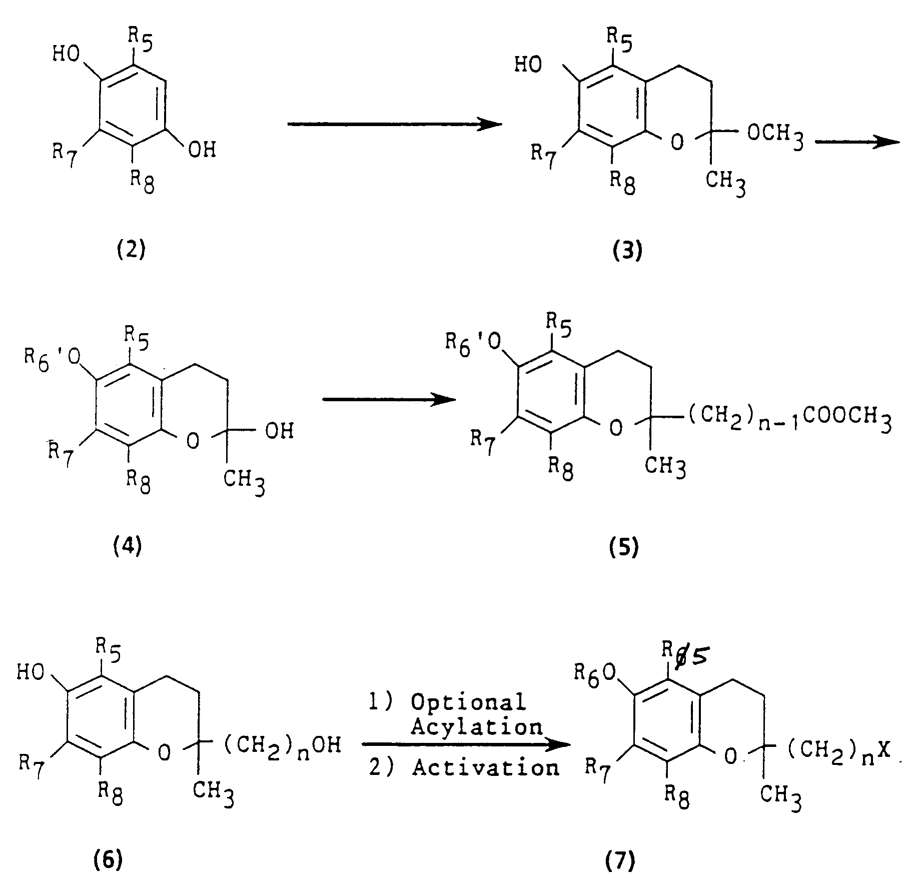

### Preparation of final compounds

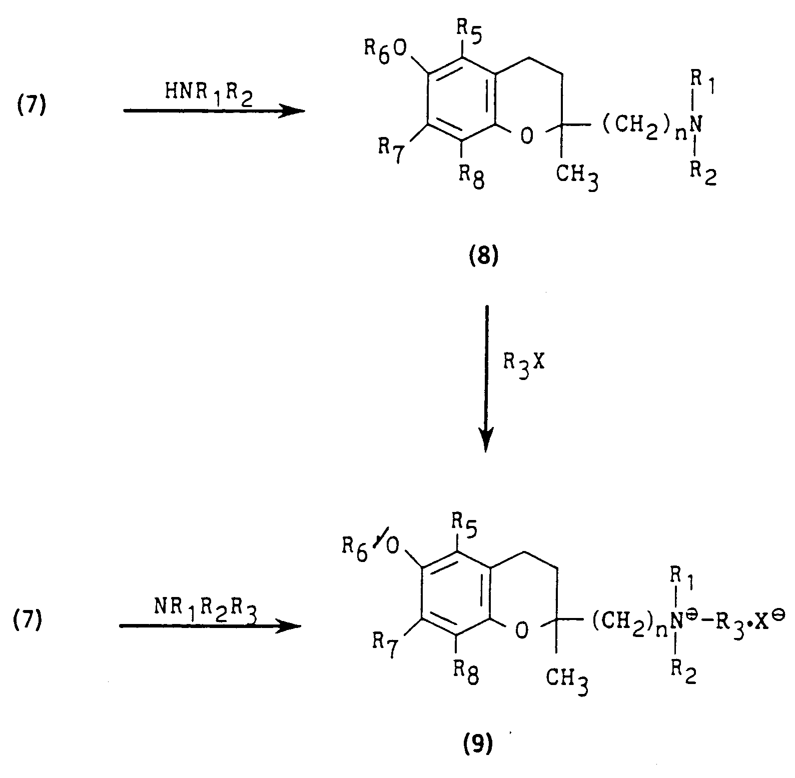

wherein n, R₁, R₂,R₃, R₅, R₆, R₇, R₈ and X are as previously defined and R₆' is -C(O)R, R being as previously defined.

The preparation of the intermediates start with the condensation of hydroquinones (2) with 3-butene-2-one in the presence of an acid, preferably sulfuric acid, the condensation being effected in methanol and trimethyl orthoformate. The so-produced dihydrobenzopyrans (3) are then sequentially subjected to acylation and hydrolysis reactions according to standard procedures to yield the hemiketals of Formula (4).
Introduction of the hydroxyalkyl moiety at the 2-position of the compounds of Formula (4) can be effected by Wittig or Horner type reactions, preferably by reaction of the compounds of Formula (4) with a trimethylphosphonoester (e.g. trimethylphosphonoacetate) to yield the esters of Formula (5) which are hydrolyzed, and then reduced (preferably with lithium aluminum hydride) to yield the alcohols of Formula (6). These alcohols may also be formed directly by an acid catalyzed condensation of the hydroquinones (2) with the appropriate vinyl diols of Formulae (10) and (11). 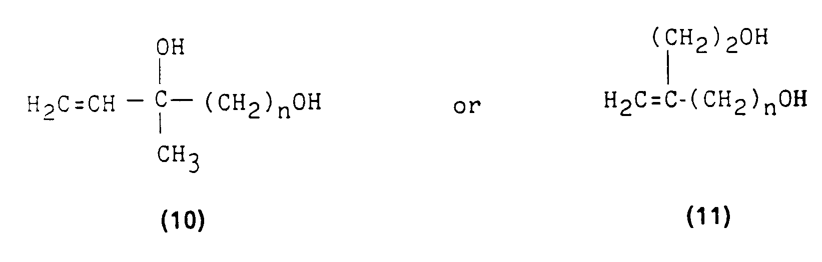
n being as defined above.

Prior to amination, the alcohols of Formula (6) are first activated by converting the 2-position hydroxyalkyl moieties to either their halides or tosylates (i.e., X is a halide or a p-toluenesulfonyl radical of the formula -OS(O)₂R₄ wherein R₄ is as previously defined) according to standard conditions such as for example reaction of the alcohols with bromotriphenyl phosphonium bromide (Ø₃PBr⁺Br⁻) obtained by reaction of triphenylphosphine with bromine in dichloromethane, or by reacting the alcohols with the appropriate sulfonyl halide (e.g., p-toluenesulfonyl chloride) in the presence of a base according to standard procedures well known in the art. The resulting activated halides (or tosylates) (7) may be converted to the desired dialkylamino or quaternaryammonium derivatives either before or after acylation of the 6-OH moiety. Standard amination procedures may be utilized to prepare the desired tertiary or quaternary amines of Formula I.
In the instance wherein it is desired to prepare the tertiary amines it is preferred to react the compounds of Formula (7) with the appropriate dialkylamine such as by contacting equimolar quantities of the reactants at temperatures of about 30°C to 90°C, with stirring, in an inert solvent, preferably dimethylformamide, said amination preferably taking place after the 6-position -ol compound has been acylated. Similarly, standard procedures well known in the art may be used in the preparation of the quaternary ammonium derivatives of Formula I. For example, reacting the activated compounds of Formula (7) with equimolar quantities of the appropriate trialkyl amine, under pressure, at temperatures of about 90°C to 150°C, in an inert solvent, preferably butanone, may be efficiently utilized. Alternatively, the quaternary ammonium derivatives may be prepared from the tertiary amines (as free bases) by reacting the tertiary amine with the appropriate alkyl halide or alkyl sulfonate (i.e., R₃X wherein X is a halide or -OS(O)₂R₄) according to standard procedures such as by heating the reactants, preferably at reflux temperatures, in a basic solvent, preferably acetonitrile. Again, it is preferred to acylate prior to amination. As stated, it is preferred to acylate the 6-position moiety prior to amination and thus the reaction schemes so-depict the preferred route of synthesis. If desired, amination may be effected prior to acylation but it is a less desirable route of synthesis.

Further, as there is an asymmetric carbon atom at the 2-position, the compounds may occur as either the R- or the S-enantiomers, or mixtures thereof. The preparation of the individual enantiomeric form may be effected by resolving the acids of Formula (5) by standard and conventional means such as, for example, via the use of diastereomeric salts with optically active amines, or alternatively, by resolving the alcohols (7) as esters with optically active acids, e.g. L-2,4-MeClC₆H₃CHMeCOOH (Me representing methyl).

The following examples will serve to illustrate the techniques and processes described herein.

### EXAMPLE 1

### 3,4-Dihydro-2-(2-bromoethyl)-2,5,7,8-tetramethyl-2H-1-benzopyran-6-ol

To 11.0 g (0.042 mol) of triphenylphosphine in 200 ml of dichloromethane is added dropwise a solution of 6.71 g (0.042 mol) of bromine in 50 ml of dichloromethane. The solution is stirred for 30 min at room temperature, then 10.0 g (0.04 mol) of 3,4-dihydro-6-hydroxy-2,5,7,8-tetramethyl-2H-1-benzopyran-2-ethanol (CAS 79907-48-5) is added. The resulting solution is refluxed for 4 hours, allowed to cool overnight, washed with a solution of 15 g of sodium carbonate in 200 ml of water, dried over anhydrous sodium sulfate, filtered and evaporated. The resulting oil is crystallized from methanol to give 9.22 g of 3,4-dihydro-2-(2-bromoethyl)-2,5,7,8-tetramethyl-2H-1-benzopyran-6-ol.

The optically active enantiomers are obtained by substituting racemic 3,4-dihydro-6-hydroxy-2,5,7,8-tetramethyl-2H-1-benzopyran-2-ethanol with enantiomer R- (CAS 94425-68-0) or S-(CAS 94425-67-9) and by following the procedures of this example for each individual isomer.

### EXAMPLE 2

### 3,4-Dihydro-2-(2-bromoethyl)-2,5,7,8-tetramethyl-2H-1-benzopyran-6-yl acetate

To a solution of 9.22 g (0.029 mol) of 3,4-dihydro-2-(2-bromoethyl)-2,5,7,8-tetramethyl-2H-1-benzopyran-6-ol in 60 ml of lutidine is added 30 ml of acetic anhydride. The resulting solution is stirred at room temperature overnight. Water (30 ml) is added and some ice to keep the temperature around 30°C, the mixture is stirred for 30 min, more water and ice are added, the resulting precipitate is collected, washed with water and dried over phosphorus pentoxide under reduced pressure to give 10.0 g of powder. Recrystallization from a mixture of ethyl ether and pentane gives 9.41 g of 3,4-dihydro-2-(2-bromoethyl)-2,5,7,8-tetramethyl-2H-1-benzopyran-6-yl acetate, m.p. 102-103°C.

### EXAMPLE 3

### 3,4-Dihydro-2-(2-dimethylaminoethyl)-2,5,7,8-tetramethyl-2H-1-benzopyran-6-ol hydrochloride

A mixture of 12.53 g of 3,4-dihydro-2-(2-bromoethyl)-2,5,7,8-tetramethyl-2H-1-benzopyran-6-ol and liquid dimethylamine in 50 ml of dimethylformamide is stirred at room temperature for 16 hours. Water is added and the product is extracted with ethyl ether. The extract is washed with water, dried over anhydrous sodium sulfate, filtered and evaporated. One equivalent of hydrochloride acid in isopropanol is added and the resulting precipitate is recrystallized twice from isopropanol/water to yield 9.44 g of the title compound, m.p. >300°C.

### EXAMPLE 4

### 3,4-Dihydro-2-(2-dimethylaminoethyl)-2,5,7,8-tetramethyl-2H-1-benzopyran-6-yl acetate

A mixture of 3.55 g (0.01 mol) of 3,4-dihydro-2-(2-bromoethyl)-2,5,7,8-tetramethyl-2H-1-benzopyran-6-yl acetate and 2.0 g of liquid dimethylamine in 50 ml of dimethylformamide is stirred at room temperature for 40 hours. Water is added and the product is extracted with ethyl acetate and ethyl ether. The extract is washed with water, dried over anhydrous sodium sulfate, filtered and evaporated. The resulting oil crystallizes from a mixture of ethyl ether and pentane to give 2.05 g of 3,4-dihydro-2-(2-dimethylaminoethyl)-2,5,7,8-tetramethyl-2H-1-benzopyran-6-yl acetate as the free base.

### EXAMPLE 5

### 3,4-Dihydro-2-(2-dimethylaminoethyl)-2,7,8-trimethyl-2H-1-benzopyran-6-ol

Following the procedure described in Examples 1-3, but using 3,4-dihydro-6-hydroxy-2,7,8-trimethyl-2H-1-benzopyran-2-ethanol (CAS 93600-70-5) as starting material, the title compound is obtained.

### EXAMPLE 6

### 3,4-Dihydro-2-(2-dimethylaminoethyl)-2,5,8-trimethyl-2H-1-benzopyran-6-ol

Following the procedure described in Example 3, but using 3,4-dihydro-6-hydroxy-2,5,8-trimethyl-2H-1-benzopyran-2-ethanol (CAS 93600-69-2) as starting material, the title compound is obtained.

### EXAMPLE 7

### 3,4-Dihydro-2-(2-dimethylaminoethyl)-2,5,7-trimethyl-2H-1-benzopyran-6-ol

Following the procedure described in Examples 1 and 3, but using 3,4-dihydro-6-hydroxy-2,5,7-trimethyl-2H-1-benzopyran-2-ethanol (CAS 93600-68-1) as starting material, the title compound is obtained.

### EXAMPLE 8

### 3,4-Dihydro-2(2-dimethylaminoethyl)-2,5,7,8-tetramethyl-6-(1,1-dimethyl-ethylcarbonyloxy)-2H-1-benzopyran

Following the procedure described in Example 2, but substituting acetic anhydride by an equimolar amount of pivaloyl chloride, 3,4-dihydro-2-(2-bromoethyl)-2,5,7,8-tetramethyl-2H-1-benzopyran-6-yl α,α-dimethylpropionate is obtained, which is then converted to the title compound by the procedure described in Example 4.

### EXAMPLE 9

### 3,4-Dihydro-3-(3-dimethylaminopropyl)-2,5,7,8-tetramethyl-2H-1-benzopyran-6-ol

Following the procedure described in Examples 1 and 3, but using 3,4-dihydro-6-hydroxy-2,5,7,8-tetramethyl-2H-1-benzopyran-2-propanol (CAS 104568-57-2) as starting material, the title compound is obtained.

### EXAMPLE 10

### 2-(3,4-Dihydro-6-hydroxy-2,5,7,8-tetramethyl-2H-1-benzopyran-2-yl)ethyl-N,N,N-trimethylammonium bromide

Step A: To 11.0 g (0.042 mol) of triphenylphosphine in 200 ml of dichloromethane is added dropwise a solution of 6.71 g (0.042 mol) of bromine in 50 01 of dichloromethane. The solution is stirred for 30 min at room temperature, then 10.0 g (0.04 mol) of 3,4-dihydro-6-hydroxy-2,5,7,8-tetramethyl-2H-1-benzopyran-2-ethanol (CAS 79907-48-5) is added. The resulting solution is refluxed for 4 hours, allowed to cool overnight, washed with a solution of 15 g of sodium carbonate in 200 ml of water, dried over anhydrous sodium sulfate, filtered and evaporated. The resulting oil is crystallized from methanol to give 9.22 g of 3,4-dihydro-2-(2-bromoethyl)-2,5,7,8-tetramethyl-2H-1-benzopyran-6-ol.
Step B: To 2.90 g of the above bromide in 80 ml of butanone, in a stainless steel bomb is added 6 g of cold (-20°C) liquid trimethylamine; the bomb is sealed and heated to 120-125°C for 60 hours with internal stirring. The bomb is cooled, opened, and its content is transferred to a flask with a solvent. The solvent is evaporated and the residue is recrystallized from ethanol to give 1.99 g of the title compound, m.p. 225°C.

The optically active enantiomers are obtained by substituting racemic 3,4-dihydro-6-hydroxy-2,5,7,8-tetramethyl-2H-1-benzopyran-2-ethanol with enantiomer R- (CAS 94425-68-0) or S-(CAS-94425-67-9) and by following the procedures of Step A and B for each individual isomer.

### EXAMPLE 11

### 2-(3,4-Dihydro-2,5,7,8-tetramethyl-6-methylcarbonyloxy-2H-1-benzopyran-2-yl)ethyl-N,N,N-trimethylammonium bromide

Step A: To a solution of 9.22 g (0.029 mol) of 3,4-dihydro-2-(2-bromoethyl)-2,5,7,8-tetramethyl-2H-1-benzopyran-6-ol in 60 ml of lutidine is added 30 ml of acetic anhydride. The resulting solution is stirred at room temperature overnight. Water (30 ml) is added and some ice to keep the temperature around 30°C, the mixture is stirred for 30 min, more water and ice are added, the resulting precipitate is collected, washed with water and dried over phosphorus pentoxide under reduced pressure to give 10.0 g of powder. Recrystallization from a mixture of ethyl ether and pentane gives 9.41 g of 3,4-dihydro-2-(2-bromoethyl)-2,5,7,8-tetramethyl-2H-1-benzopyran-6-yl acetate, m.p. 102-103°C.
Step B: To 6.71 g (0.019 mol) of this acetate in 80 ml of butanone is added about 10 g of cold (-20°C) liquid trimethylamine. The mixture is stirred and heated to 100-105°C in a sealed stainless steel vessel for 60 hours, the solvent is evaporated and the resulting solid is recrystallized twice from ethanol to give 5.18 g of the title compound, m.p. 285°C.

### EXAMPLE 12

### 2-(3,4-Dihydro-6-hydroxy-2,7,8-trimethyl-2H-1-benzopyran-2-yl)ethyl-N,N,N-trimethylammonium bromide

Following the procedure described in Example 10, but using 3,4-dihydro-6-hydroxy-2,7,8-trimethyl-2H-1-benzopyran-2-ethanol (CAS 93600-70-5) as starting material, the title compound is obtained.

### EXAMPLE 13

### 2-(3,4-Dihydro-6-hydroxy-2,5,8-trimethyl-2H-1-benzopyran-2-yl)ethyl-N,N,N-trimethylammonium bromide

Following the procedure described in Example 10, but using 3,4-dihydro-6-hydroxy-2,5,8-trimethyl-2H-1-benzopyran-2-ethanol (CAS 93600-69-2) as starting material, the title compound is obtained.

### EXAMPLE 14

### 2-(3,4-Dihydro-6-hydroxy-2,5,7-trimethyl-2H-1-benzopyran-2-yl)ethyl-N,N,N-trimethylammonium bromide

Following the procedure described in Example 10, but using 3,4-dihydro-6-hydroxy-2,5,7-trimethyl-2H-1-benzopyran-2-ethanol (CAS 93600-68-1) as starting material, the title compound is obtained.

### EXAMPLE 15

### 2-[3,4-Dihydro-2,5,7,8-tetramethyl-6-(1,1-dimethyl-ethylcarbonyloxy)-2H-1-benzopyran-2-yl]ethyl-N,N,N-trimethylammonium bromide

Following the procedure described in Example 11, but substituting acetic anhydride by an equimolar amount of pivaloyl chloride, 3,4-dihydro-2-(2-bromoethyl)-2,5,7,8-tetramethyl-2H-1-benzopyranyl α,α-dimethylpropionate is obtained, which is then converted to the title compound by either the procedure described in Example 11 or that described in Example 18.

### EXAMPLE 16

### 3-(3,4-Dihydro-6-hydroxy-2,5,7,8-tetramethyl-2H-1-benzopyran-2-yl)propyl-N,N,N-trimethylammonium bromide

Following the procedure described in Example 10, but using 3,4-dihydro-6-hydroxy-2,5,7,8-tetramethyl-2H-1-benzopyran-2-propanol (CAS 104568-57-2) as starting material, the title compound is obtained.

### EXAMPLE 17

### 2-(3,4-Dihydro-2,5,7,8-tetramethyl-6-methylcarbonyloxy-2H-1-benzopyran-2-yl)ethyl-N,N,N-trimethylammonium p-toluenesulfonate

A mixture of 3.55 g (0.01 mole) of 3,4-dihydro-2-(2-bromoethyl)-2,5,7,8-tetramethyl-2H-1-benzopyran-6-yl acetate and 2.0 g of liquid dimethylamine in 50 ml of dimethylformamide is stirred at room temperature for 40 hours. Water is added and the product is extracted with ethyl acetate and ethyl ether. The extract is washed with water, dried over anhydrous sodium sulfate, filtered and evaporated. The resulting oil crystallizes from a mixture of ethyl ether and pentane to give 2.05 g of 3,4-dihydro-2-(2-dimethylaminoethyl)-2,5,7,8-tetramethyl-2H-1-benzopyran-6-yl acetate as the free base.

A solution of 319 mg of the above base and 204 mg (10% excess) of methyl p-toluenesulfonate in 10 ml of acetonitrile is refluxed for 3 hours. The solvent is evaporated and the resulting oil is triturated with ethyl ether. The resulting solid is recrystallized from ethyl acetate to give 302 mg of the title compound, m.p. 77°C.

### EXAMPLE 18

### N-Butyl-N-[2-(3,4-dihydro-2,5,7,8-tetramethyl-6-methylcarbonyloxy-2H-1-benzopyran-2-yl)ethyl]-N,N-dimethylammonium chloride

Following the procedure described in Example 17, but substituting methyl p-toluenesulfonate by 1-chlorobutane sulfonate, the title compound is obtained.

Having described the scope of the compounds of this invention as well as the generic and specific methods for preparing said compounds, the following information describes the utility, and the methods therefor, of the compounds of this invention.

When the blood supply to parts of the heart muscle is blocked, a myocardial infarct (heart attack) results and the deprived muscle tissue dies with the result of permanent heart damage. If the blood supply can be re-established within hours after infarction, the heart muscle tissue remains viable and permanent damage can be reduced. This can be accomplished by surgical as well as pharmacologic (thrombolysis) procedures and these processes are known as reperfusion.

Reperfusion is now widely and successfully applied and it has been claimed that fatalities due to myocardial infarction can be reduced by 20-30%. However, reperfusion also poses problems. Oxygen-deprived (ischemic) tissue finds itself in an abnormal state and is vulnerable when suddenly exposed to oxygen-rich blood. This has been termed the "oxygen paradox" and leads to reperfusion damage in the form of cell death. It has been postulated that this damage is due to oxygen-derived free radicals and, in particular, to the superoxide radical, O₂⁻. Evidence for this hypothesis has been obtained in animal experiments. B.R. Lucchesi and coworkers showed that the enzyme superoxide dismutase, as well as the free radical scavenger N-(mercaptopropionyl)glycine reduce canine myocardial reperfusion injury (Cir. Res., 1984, 54, 277-285; J. Cardiovasc. Pharmacol., 1986, 8, 978-88; Fed. Proc., 1987, 46, 2413-21).

Vitamin E, i.e., α-tocopherol, a well known compound of the formula 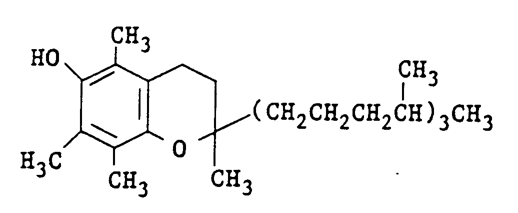
is a natural anti-oxidant that reacts with oxygen-derived free radicals as well as hydrogen peroxide. It has been shown that it is intercalated in lipid membranes and that its biological function is to protect biomembranes against oxidative attack. The anti-oxidant 3,4-dihydro-2,5,7,8-tetramethyl-2H-2-benzopyran-6-ol moiety of α-tocopherol is constantly regenerated by the ubiquitous cytosolic redox systems and for all practical purposes is a permanent membrane constituent that is constantly regenerated.

The compounds of this invention also possess a related or similar 3,4-dihydro-2,5,7,8-tetraalkyl-2H-1-benzopyran-2-yl moiety, but the 2-position lipophylic moiety of the α-tocopherol molecule, which is thought to be responsible for its ubiquitous incorporation into biomembranes, is replaced with a hydrophylic moiety to impart a greater affinity for cardiac tissue. Thus, the compounds of this invention are also useful as pharmacologic antioxidants and free radical scavengers and, in particular, as scavengers of superoxide anion radical O₂⁻. They can be therapeutically employed where reperfusion damage due to oxygen-derived free radicals and hydrogen peroxide causes cell death in tissues. This situation arises when total or partial blockade of blood supply to tissues is removed, either spontaneously (transient ischemia) or by pharmacologic or surgical intervention (thrombolysis, angioplasty, by-pass, organ transplant and the like). Tissues subjected to transient ischemia or reperfusion in various disease states, or by their medical treatment, are those of heart, lung, kidney, pancreas and brain. In particular, the now rapidly increasing practice of pharmacologic thrombolysis, also known as reperfusion, after coronary infarct and stroke, will benefit by prior or concomitant administration of a free radical scavenger such as the compounds of this invention. Similarly, surgical interventions, such as percutaneous transluminal coronary angioplasty, where a dilating balloon is used to increase the luminal diameter in severely occluded atherosclerotic vessels, coronary by-pass operations, and organ transplant surgery create conditions where reperfusion damage due to oxygen-derived radicals takes place and can be reduced by scavengers. Transient ischemia is one of the causative factors that lead to angina pectoris, and thus the compounds of this invention are also useful as antianginal agents.

The process of inflammation is also known to involve the release of superoxide radicals from phagocytic cells which cause some of the symptoms of rheumatoid arthritis and a free radical scavenger, such as the compounds of this invention, is also useful in the treatment of this disease. The compounds may also be useful in the treatment of cancers and aging since oxygen-derived free radicals have been identified among causative factors. For reviews, see B. Halliwell and C. Gutteridge, Biochem. J., 1984, 219, 1-14; TINS 1985, 22-6.

*In vitro* and *in vivo* activity for the compounds of this invention may be demonstrated by the use of standard assays which demonstrate the free radical scavenging property, affinity for cardiac tissue and cardioprotective properties.

### Free Radical Scavenging Property

2-(3,4-dihydro-6-hydroxy-2,5,7,8-tetramethyl-2H-1-benzopyran-2-yl)ethyl-N,N,N-trimethylammonium bromide, described in Example 10, was evaluated for its ability to reduce superoxide radicals in an assay described by H.P. Misra and J. Fridovich, 1972, J. Biol. Chem. 247, 3170-5. In this assay, the rate of autoxidation of 1-epinephrine (1 x 10⁻⁴M) in the presence of EDTA (1 x 10⁻⁴M) at 30°C is followed spectrophotometrically following the increase of absorbance at 480 nm. 50% inhibition of the autoxidation rate (ID₅₀) was obtained at 138 ± 14 »M of the test compound.

For comparison, 3,4-dihydro-6-hydroxy-2,5,7,8-tetramethyl-2H-1-benzopyran-2-carboxylic acid (CAS 53188-07-1) gave ID₅₀ = 105 ± 8 »M and d,3-penicillamine (CAS 52-67-5) ID₅₀ = 73 »M in parallel experiments. Misra and Fridovich report that bovine superoxide dismutase gave 50% inhibition at 46 mg per ml.

### Affinity for cardiac tissue

Following the procedure described in Example 17, but using ¹⁴C-methyl p-toluenesulfonate in place of "cold material"_{,} ¹⁴C-labelled 2-(3,4-dihydro-2,5,7,8-tetramethyl-6-methylcarbonyloxy-2H-1-benzopyran-2-yl)ethyl-N,N,N-tri-¹⁴C-methylammonium p-toluenesulfonate was synthesized. The preparation was diluted with "cold material" to 11.74 »Ci/mg base.

24 rats were given 0.91 mg base/kg by intravenous route (by canula into the jugular vein). The animals were sacrificed in groups of 3 at the time periods indicated in Table I. Immediately before sacrifice a 0.5 ml blood sample was taken from an indwelling catheter in the carotid artery. The hearts were removed, blotted and weighed. The samples of tissue and blood were solubilized with Luma Solve (a quaternary ammonium hydroxide preparation) and radioactivity was determined in a scintillation counter. The results are shown in Table I.

**TABLE I**

| Time after administration | ng equiv/g heart | ng equiv/ml blood | Ratio heart/blood |
|---|---|---|---|
| 5 min | 1.591 ± 0.22 | 0.434 ± 0.351 | 3.05 ± 1.46 |
| 15 min | 1.728 ± 0.422 | 0.590 ± 0.295 | 6.90 ± 8.34 |
| 30 min | 1.905 ± 0.278 | 0.124 ± 0.032 | 17.33 ± 5.42 |
| 1 hour | 2.092 ± 0.406 | 0.096 ± 0.008 | 20.09 ± 2.54 |
| 2 hours | 1.395 ± 0.042 | 0.086 ± 0.041 | 21.90 ± 3.56 |
| 4 hours | 1.253 ± 0.248 | 0.054 ± 0.013 | 27.15 ± 4.30 |
| 6 hours | 0.863 ± 0.067 | 0.048 ± 0.007 | 18.62 ± 2.77 |
| 8 hours | 0.652 ± 0.116 | 0.048 ± 0.012 | 15.12 ± 2.69 |

It can be seen that the test compound has a marked affinity for heart tissue. The ratio of radioactivity per g of heart tissue to radioactivity per ml of blood increased with time to reach a maximum of 27.15 at 4 h. Even after 8 h the concentration of drug in heart tissue remains elevated (0.65 »g equiv/g).

### Cardioprotective effects

2-(3,4-Dihydro-2,5,7,8-tetramethyl-6-methylcarbonyloxy-2H-1-benzopyran-2-yl)ethyl-N,N,N-trimethylammonium bromide was evaluated in ligation-induced infarcted and reperfused rats as follows. One of two groups of rats was infused intravenously with a solution of test compound in saline at a rate of 2.3 ml/h (10 mg/kg/h). The control group was infused with saline at the same rate. After 10 min of drug infusion, coronary arteries were ligated surgically for 60 min, ligation was loosened to allow reperfusion for 30 min. The ligation was retied and a dye (Evans Blue) was injected. The animals were sacrificed and the heart ventricles were removed and weighed. The unstained tissue was dissected and weighed; this represents the "area at risk", i.e. the area that was deprived of blood supply by ligation. To determine the infarcted area, the tissue was incubated with 2,3,5-triphenyltetrazolium chloride. Infarcted tissue became light colored and could be dissected and weighed. Thus, for each rat that survived the ligation a measurement of "area at risk" and of "infarcted area" was obtained and the ratio was calculated as given in Tables II and III.

**TABLE II**

| Control Group | | | | |
|---|---|---|---|---|
| Rat No. | Total Ventr. wt., g. | Tissue at risk, mg | Infarcted tissue, mg | Ratio % |
| 1 | 1.445 | 196 | 51 | 25.7 |
| 2 | 1.362 | 383 | 200 | 52.3 |
| 3 | 1.428 | 277 | 152 | 54.6 |
| 4 | 1.470 | 244 | 232 | 95.2 |
| 5 | 1.359 | 272 | 323 | 119 |
| 6 | 1.530 | 452 | 432 | 95.6 |
| 7 | 1.220 | 303 | 339 | 112 |
| 8 | 1.520 | 188 | 173 | 93.5 |
| 9 | 1.133 | 78 | 80 | 102.6 |
| average | | | | 83.31 |
| standard deviation | | | | 31.72 |

**TABLE III**

| Treated Group (10 mg/kg/h) | | | | |
|---|---|---|---|---|
| Rat No. | Total Ventr. wt., g. | Tissue at risk, mg | Infarcted tissue, mg | Ratio % |
| 1 | 1.381 | 307 | 299 | 97.5 |
| 2 | 1.237 | 255 | 39 | 15.5 |
| 3 | 1.336 | 176 | 33 | 18.9 |
| 4 | 1.197 | 381 | 247 | 64.8 |
| 5 | 1.321 | 310 | 193 | 62.1 |
| 6 | 1.203 | 207 | 134 | 64.5 |
| 7 | 1.619 | 395 | 123 | 31.2 |
| 8 | 0.979 | 229 | 157 | 68.4 |
| 9 | 1.194 | 50 | 23 | 45.2 |
| 10 | 1.342 | 285 | 27 | 9.4 |
| average | | | | 47.75 |
| standard deviation | | | | 28.48 |

It can be seen that the untreated group gave a ratio of 83.3%. and the treated group gave a ratio of 47.8%, i.e. treatment resulted in 42.7% reduction of infarction. The result is statistically significant (ANOVA) at p = 0.02.

### Cardioprotective effects

3,4-Dihydro-2-(2-dimethylaminoethyl)-2,5,7,8-tetramethyl-2H-1-benzopyran-6-yl acetate hydrochloride was evaluated in ligation-induced infarcted and reperfused rats as follows. One of two groups of rats was infused intravenously with a solution of test compound in saline at a rate of 2.3 ml/h (10 mg/kg/h). The control group was infused with saline at the same rate. After 10 min of drug infusion, coronary arteries were ligated surgically for 60 min, ligation was loosened to allow reperfusion for 30 min. The ligation was retied and a dye (Evans Blue) was injected. The animals were sacrificied and the heart ventricals were removed and weighed. The unstained tissue was dissected and weighed; this represents the "area at risk", i.e. the area that was deprived of blood supply by ligation. To determine the infarcted area, the tissue was incubated with 2,3,5-triphenyltetrazolium chloride. Infarcted tissue became light colored and was dissected and weighed. Thus, for each rat that survived the ligation a measurement of "area at risk" and of "infarcted area" was obtained and the ratio was calculated as shown in the following tables.

**TABLE IV**

| Control Group | | | | |
|---|---|---|---|---|
| Rat No. | Total ventr. wt., g. | Tissue at risk mg | Infarcted tissue mg | ratio % |
| 1 | 1.203 | 410 | 403 | 98.3 |
| 2 | 1.521 | 459 | 401 | 87.4 |
| 3 | 1.090 | 249 | 245 | 98.4 |
| 4 | 1.142 | 180 | 154 | 85 |
| 5 | 0.973 | 327 | 78 | 24 |
| 6 | 1.054 | 125 | 113 | 90 |
| 7 | 0.784 | 465 | 321 | 89 |
| Average | | | | 78.9 |
| Standard deviation | | | | 26.2 |

**TABLE V**

| Treated Group | | | | |
|---|---|---|---|---|
| Rat No. | Total ventr. wt., g. | Tissue at risk mg | Infarcted tissue mg | ratio % |
| 1 | 1.193 | 478 | 337 | 70.1 |
| 2 | 1.046 | 200 | 80 | 40.3 |
| 3 | 1.383 | 513 | 51 | 10 |
| 4 | 1.078 | 416 | 346 | 83.2 |
| 5 | 1.0 | 457 | 18 | 4 |
| 6 | 0.95 | 411 | 294 | 71.6 |
| 7 | 0.830 | 292 | 51 | 17.5 |
| 8 | 0.895 | 487 | 34 | 7.0 |
| Average | | | | 38.0 |
| Standard deviation | | | | 32.9 |

It can be seen that the untreated group gave a ratio of 78.9% and the treated group gave a ratio of 38.0%, i.e., treatment resulted in 48.2% reduction of infarction. The result is statistically significant (ANOVA) at p < 0.02.

Most preferably, the compounds are administered intravenously particularly under crisis situations wherein it is essential that the therapeutic agent be gotten to its site of action as quickly as possible, such as in those emergency conditions caused by coronary infarction, stroke and surgical interventions, conditions which can cause severe reperfusion damage. Preferably, the compounds of this invention are administered intravenously wherein Q is a dialkylamino moiety, and orally in those instances wherein Q is a quaternary ammonium salt.

The compounds of this invention can be utilized both prophylactically and therapeutically. The amount of active ingredient for therapeutic administration can vary over a wide range and is dependent upon such factors as the species of mammal to be treated, its age, health, sex, weight, nature and the severity of the condition being treated. Generally, a therapeutically effective amount of the active ingredient to be administered will range from about 0.1 mg/kg to 50 mg/kg of body weight per day. For prophylactic administration, corresponding lower doses can be utilized.

The compounds of this invention also can be orally administered, preferably using more active ingredient per day than when parenterally administered, preferably taking divided doses 3 to 4 times per day. Preferably, enteral administration in post "crisis" situations, particularly after release from hospitalized conditions. The compounds can be used in standard dosage unit forms such as tablets, capsules, dragees, lozenges, elixirs, emulsions, suspensions, and in cases wherein topical application is preferred by suppository or sub-lingual administration. Tablets and capsules containing from 100 to 400 mg of active ingredient are preferred modes of enteral administration. Of course, in the treatment of inflammation the preferred method of administration is by depot injection directly to the situs of the inflammation area with follow-up enteral means of administration.

In preparing solid dose forms such as tablets, the active ingredient is generally blended with conventional pharmaceutical carriers or excipients such as gelatin, various starches, lactose, calcium phosphate or powdered sugar. The term pharmaceutical carrier as used herein also includes lubricants employed to improve the flow of tablet granulations and which prevent adhesion of tablet material to the surfaces of tablet dies and punches. Suitable lubricants include, for example, talc, stearic acid, calcium stearate, magnesium stearate and zinc stearate. Also included within the definition of a pharmaceutical carrier as used herein, are disintegrating agents added to assist the breakup and dissolution of tablets following administration, as well as coloring and/or flavoring agents to enhance the aesthetic qualities of the tablets and make them more acceptable to the patient.

Suitable liquid excipients for the preparation of liquid dosage unit forms include water and alcohols such as ethanol, benzyl alcohol and the polyethylene glycols, either with or without the addition of a surfactant. In general, the preferred liquid excipients, particularly for injectable preparations, include water, physiological and saline solutions, dextrose and glycol solutions such as an aqueous propylene glycol or polyethylene glycol solutions. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to 15% by weight. The surfactant can be a single component having the above-identified HLB, or a mixture of two or more components having the desired HLB. Illustrative of surfactants useful in parenteral formulations are the class of polyoxyethylene sorbitan fatty acid esters as, for example, sorbitan monooleate and the high molecular weight adducts of ethylene oxide with a hydrophobic base, formed by the condensation of propylene oxide with propylene glycol. In certain topical and parenteral preparations, various oils can be utilized as carriers or excipients. Illustrative of such oils are mineral oils, glyceride oils such as lard oil, cod liver oil, peanut oil, sesame oil, corn oil and soybean oil. For insoluble compounds, suspending agents may be added as well as agents to control the viscosity, as for example, magnesium aluminum silicate or carboxymethylcellulose. In addition to these excipients, buffers, preservatives and emulsifying agents may also be added.

Of course, as is true in most instances wherein certain classes of chemical compounds have been found to have beneficial therapeutic end-use applications, certain sub-generic groups and certain specific compounds are preferred. In this instance the preferred compounds of Formula I are those wherein R₅, R₇ and R₈ are methyl; wherein R₆ is H, formyl, methyl carbonyl, t-butylcarbonyl, ethylcarbonyl, propylcarbonyl, pentylcarbonyl; wherein n is 2 (representing an ethylene moiety) and the substituents attached to the nitrogen atom are methyl. Preferred specific compounds are those compounds comprised of the foregoing preferred groups, particularly 2-(3,4-dihydro-6-hydroxy-2,5,7,8-tetramethyl-2H-1-benzopyran-2-yl)ethyl-N,N,N-trimethylammonium bromide, 2-(3,4-dihydro-2,5,7,8-tetramethyl-6-methylcarbonyloxy-2H-1-benzopyran-2-yl)ethyl-N,N,N-trimethylammonium bromide, 3,4-dihydro-2-(2-dimethylaminoethyl)-2,5,7,8-tetramethyl-2H-1-benzopyran-6-ol hydrochloride, 3,4-dihydro-2-(2-dimethylaminoethyl)-2,5,7,8-tetramethyl-2H-1-benzopyran-6-yl acetate hydrochloride, 3,4-dihydro-2-(2-dimethylaminoethyl)-2,5,7-trimethyl-2H-1-benzopyran-6-ol and 3,4-dihydro-2(2-dimethylaminoethyl)-2,5,7,8-tetramethyl-6-(1,1-dimethylethylcarbonyloxy)-2H-1-benzopyran.

## Claims

1. A compound of the formula 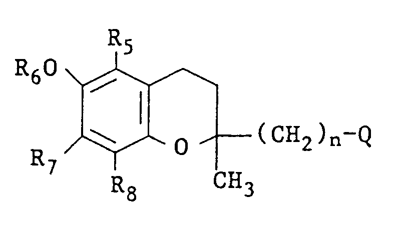 the (R) and (S) enantiomers and racemic mixtures thereof, and the pharmaceutically acceptable salts thereof wherein
Q is N^{⊕}R₁R₂R₃·X^{⊖},
X is a halide or OS(O)₂R₄, with R₄ being H, C₁₋₆ alkyl, aryl or aralkyl,
R₁, R₂ and R₃ each individually are a C₁₋₆ lower alkyl,
R₅ is H or C₁₋₆ alkyl,
R₆ is H or -C(O)R, R being H or C₁₋₉ alkyl,
R₇ is H or C₁₋₆ alkyl,
R₈ is H or C₁₋₆ alkyl and n is an integer of 1 to 6.

2. A compound of Claim 1 wherein R₅, R₇ and R₈ are methyl.

3. A compound of Claim 1 wherein R₁, R₂ and R₃ are methyl.

4. A compound of Claim 1 wherein X is chloro, bromo or p-toluenesulfonyl.

5. A compound of Claim 1 wherein R₆ is H.

6. A compound of Claim 1 wherein R₆ is RC(O)-.

7. A compound of Claim 1 wherein n is 2.

8. A compound of Claim 7 wherein R₁, R₂, R₃, R₅, R₇ and R₈ are methyl.

9. A compound of Claim 8 wherein R₆ is H.

10. A compound of Claim 9 wherein X is chloro, bromo or p-toluenesulfonyl.

11. A compound of Claim 8 wherein R₆ is RC(O)-.

12. A compound of Claim 11 wherein X is chloro, bromo or p-toluenesulfonyl.

13. A process for preparing compounds of the formula 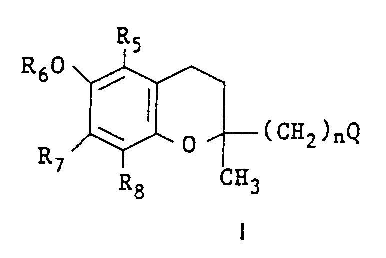 wherein Q is N^{⊕}-R₁R₂R₃·X^{⊖},
R₅, R₇ and R₈ are H or C₁₋₆ alkyl,
R₁, R₂ and R₃ are C₁₋₆ alkyl,
X is a halide or O-S(O)₂R₄, R₄ being H, C₁₋₆ alkyl, aryl or aralkyl,
R₆ is H, R-C(O), R being H or C₁₋₉ alkyl, and n is an integer 1 to 6 which comprises reactions:
(1) effecting an amination on a compound of the formula 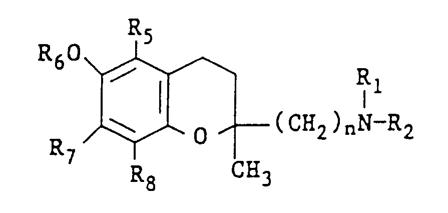 by reaction with a compound of formula R₃X,
(2) effecting an amination on a compound of the formula 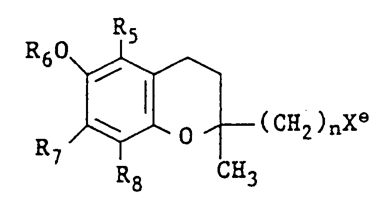 with a dialkylamine or trialkylamine of the formulae HNR₁R₂ or NR₁R₂R₃.

14. The compounds according to claim 1 to 12 for use as pharmaceutically active compounds.

15. Use of a compound of the formula 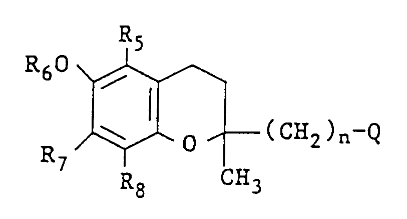 the (R) and (S) enantiomers and racemic mixtures thereof, and the pharmaceutically acceptable salts thereof wherein
Q is NR₁R₂,
X is a halide or OS(O)₂R₄, with R₄ being H, C₁₋₆ alkyl, aryl or aralkyl,
R₁ and R₂ each individually are a C₁₋₆ lower alkyl,
R₅ is H or C₁₋₆ alkyl,
R₆ is H or -C(O)R, R being H or C₁₋₉ alkyl,
R₇ is H or C₁₋₆ alkyl,
R₈ is H or C₁₋₆ alkyl and n is an integer of 1 to 6, for the preparation of a drug having cardio protective properties.

## Patentansprüche

1. Verbindung der Formel 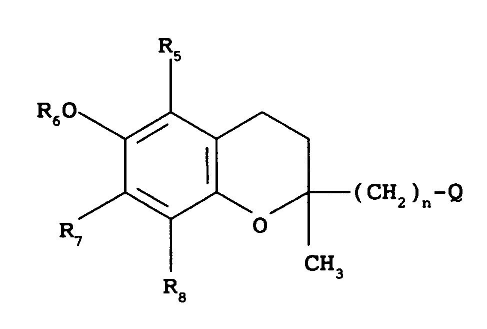 die (R)- und (S)-Enantiomere und racemische Gemische davon und pharmazeutisch verträgliche Salze davon, in der
Q ein Rest N^{⊕}R₁R₂R₃·X^{⊖} ist,
X ein Halogenatom oder ein Rest OS(O)₂R₄ ist, wobei R₄ ein Wasserstoffatom, ein C₁₋₆ Alkyl-, Aryl- oder Aralkylrest ist,
R₁, R₂ und R₃ jeweils einzeln einen C₁₋₆-Niederalkylrest bedeuten,
R₅ ein Wasserstoffatom oder ein C₁₋₆-Alkylrest ist,
R₆ ein Wasserstoffatom oder ein -C(O)R-Rest ist, wobei R ein Wasserstoffatom oder ein C₁₋₉-Alkylrest ist,
R₇ ein Wasserstoffatom oder ein C₁₋₆-Alkylrest ist,
R₈ ein Wasserstoffatom oder ein C₁₋₆-Alkylrest ist und n eine ganze Zahl von 1 bis 6 ist.

2. Verbindung nach Anspruch 1, in der R₅, R₇ und R₈ Methylgruppen sind.

3. Verbindung nach Anspruch 1, in der R₁, R₂ und R₃ Methylgruppen sind.

4. Verbindung nach Anspruch 1, in der X ein Chloratom, Bromatom oder eine p-Toluolsulfonylgruppe ist.

5. Verbindung nach Anspruch 1, in der R₆ ein Wasserstoffatom ist.

6. Verbindung nach Anspruch 1, in der R₆ ein Rest RC(O)- ist.

7. Verbindung nach Anspruch 1, in der n 2 ist.

8. Verbindung nach Anspruch 7, in der R₁, R₂, R₃, R₅, R₇ und R₈ Methylgruppen sind.

9. Verbindung nach Anspruch 8, in der R₆ ein Wasserstoffatom ist.

10. Verbindung nach Anspruch 9, in der X ein Chloratom, Bromatom oder eine p-Toluolsulfonylgruppe ist.

11. Verbindung nach Anspruch 8, in der R₆ ein Rest RC(O)- ist.

12. Verbindung nach Anspruch 11, in der X ein Chloratom, Bromatom oder eine p-Toluolsulfonylgruppe ist.

13. Verfahren zur Herstellung von Verbindungen der Formel 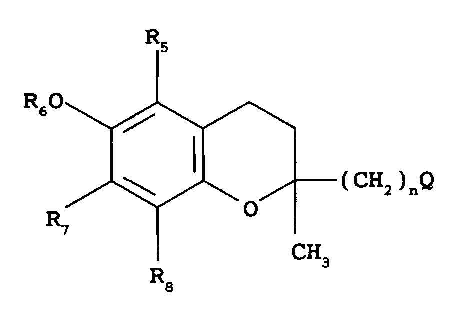 in der Q ein Rest N^{⊕}R₁R₂R₃·X^{⊖} ist,
R₅, R₇ und R₈ Wasserstoffatome oder C₁₋₆-Alkylreste sind,
R₁, R₂ und R₃ C₁₋₆-Alkylreste sind,
X ein Halogenatom oder ein Rest O-S(O)₂R₄ ist, wobei R₄ ein Wasserstoffatom, ein C₁₋₆-Alkyl-, Aryl- oder Aralkylrest ist,
R₆ ein Wasserstoffatom oder ein Rest R-C(O) ist, wobei R ein Wasserstoffatom oder ein C₁₋₉-Alkylrest ist und n eine ganze Zahl von 1 bis 6 bedeutet, das die Umsetzungen einschließt :
(1) Aminierung einer Verbindung der Formel 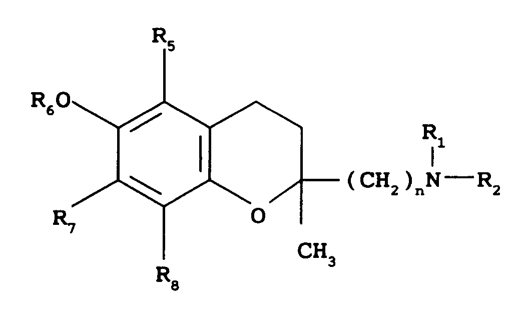 durch Umsetzung mit einer Verbindung der Formel R₃X,
(2) Aminierung einer Verbindung der Formel 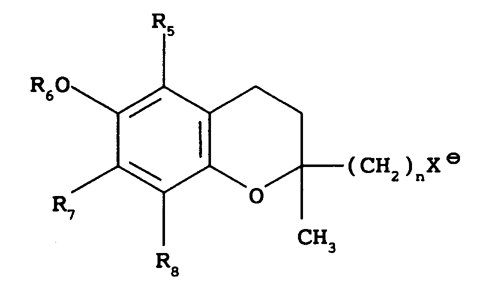 mit einem Dialkylamin oder Trialkylamin der Formeln HNR₁R₂ oder NR₁R₂R₃.

14. Verbindungen nach den Ansprüchen 1 bis 12 zur Verwendung als pharmazeutisch wirksame Verbindungen.

15. Verwendung einer Verbindung der Formel 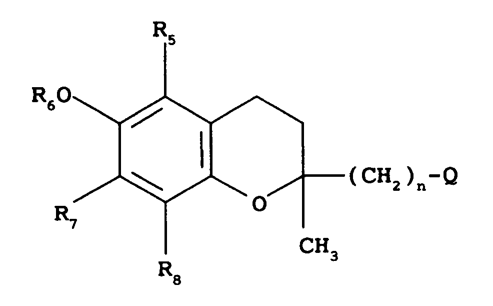 der (R)- und (S)-Enantiomeren und racemischen Gemische davon und der pharmazeutisch verträglichen Salze davon, in der
Q ein Rest NR₁R₂ ist,
X ein Halogenatom oder ein Rest OS(O)₂R₄ ist, wobei R₄ ein Wasserstoffatom, ein C₁₋₆-Alkyl-, Aryl- oder Aralkylrest ist,
R₁ und R₂ jeweils einzeln einen C₁₋₆-Niederalkylrest bedeuten,
R₅ ein Wasserstoffatom oder ein C₁₋₆-Alkylrest ist,
R₆ ein Wasserstoffatom oder ein -C(O)R-Rest ist, wobei R ein Wasserstoffatom oder ein C₁₋₉-Alkylrest ist,
R₇ ein Wasserstoffatom oder ein C₁₋₆-Alkylrest ist,
R₈ ein Wasserstoffatom oder ein C₁₋₆-Alkylrest ist und n eine ganze Zahl von 1 bis 6 ist, zur Herstellung eines Arzneimittels mit herzschützenden Eigenschaften.

## Revendications

1. Composé de formule : 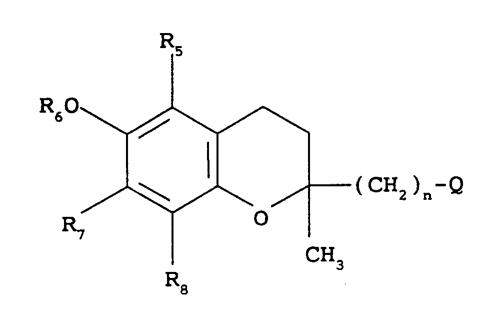 ses enantiomères (R) et (S) et ses mélanges racémiques, et ses sels pharmaceutiquement acceptables dans lesquels :
Q est N^{⊕}R₁R₂R₃·X^{⊖},
X est un halogénure ou OS(O)₂R₄, R₄ étant H, un alkyle en C₁-C₆, un aryle ou un aralkyle,
R₁, R₂ et R₃ représentent indépendamment un alkyle inférieur en C₁-C₆,
R₅ est H ou un alkyle en C₁-C₆,
R₆ est H ou un -C(O)R, R étant H ou un alkyle en C₁₋C₉,
R₇ est H ou un alkyle en C₁-C₆,
R₈ est H ou un alkyle en C₁-C₆ et n est un entier de 1 à 6.

2. Composé selon la revendication 1, dans lequel R₅, R₇ et R₈ sont le méthyle.

3. Composé selon la revendication 1, dans lequel R₁, R₂ et R₃ sont le méthyle.

4. Composé selon la revendication 1, dans lequel X est le chloro, bromo ou paratoluènesulfonyle.

5. Composé selon la revendication 1, dans lequel R₆ est H.

6. Composé selon la revendication 1, dans lequel R₆ est RC(O)-.

7. Composé selon la revendication 1, dans lequel n est 2.

8. Composé selon la revendication 7, dans lequel R₁, R₂, R₃, R₅, R₇ et R₈ sont le méthyle.

9. Composé selon la revendication 8, dans lequel R₆ est H.

10. Composé selon la revendication 9, dans lequel H est le chloro, bromo ou paratoluènesulfonyle.

11. Composé selon la revendication 8, dans lequel R₆ est RC(O)-.

12. Composé selon la revendication 11, dans lequel X est le chloro, bromo ou paratoluènesulfonyle.

13. Procédé de préparation de composés de formule : 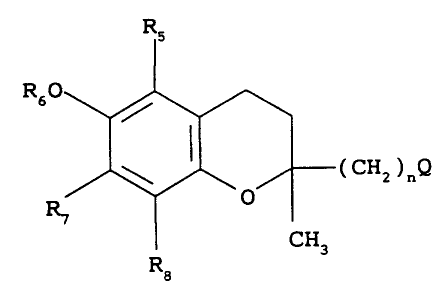 dans laquelle Q est N^{⊕}-R₁R₂R₃·X^{⊖},
R₅, R₇ et R₈ sont H ou un alkyle en C₁-C₆,
R₁, R₂ et R₃ sont un alkyle en C₁-C₆,
X est un halogénure ou O-S(O)₂R₄, R₄ étant H, un alkyle en C₁-C₆, un aryle ou un aralkyle,
R₆ est H, R-C(O), R étant H ou un alkyle en C₁-C₉, et
n est un entier de 1 à 6 qui comprend les réactions suivantes :
(1) amination d'un composé de formule : 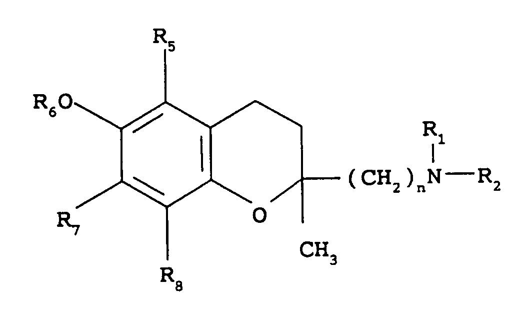 par réaction avec un composé de formule R₃X;
(2) amination d'un composé de formule : 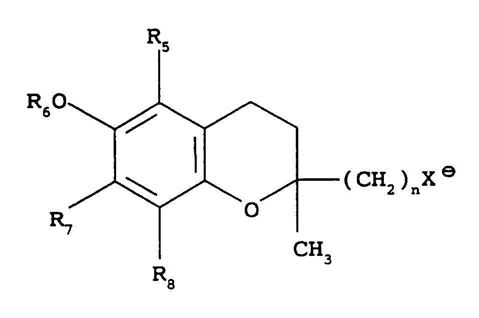 avec une dialkylamine ou une trialkylamine de formules HNR₁R₂ ou NR₁R₂R₃.

14. Composés selon les revendications 1 à 12 pour l'utilisation en tant que composés pharmaceutiquement actifs.

15. Utilisation d'un composé de formule : 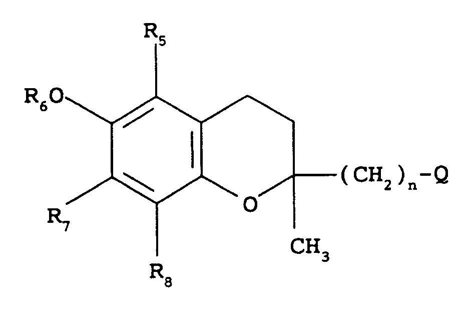 ses enantiomères (R) et (S) et ses mélanges racémiques, et ses sels pharmaceutiquement acceptables dans lesquels :
Q est NR₁R₂,
X est un halogénure ou (O)₂R₄, R₄ étant H, un alkyle en C₁-C₆, un aryle ou un aralkyle,
R₁ et R₂ représentent indépendamment un alkyle inférieur en C₁-C₆,
R₅ est H ou un alkyle en C₁-C₆,
R₆ est H ou un -C(O)R, R étant H ou un alkyle en C₁-C₉,
R₇ est H ou un alkyle en C₁-C₆,
R₈ est H ou un alkyle en C₁-C₆ et n est un entier de 1 à 6, pour préparer un
médicament qui a des propriétés cardioprotectrices.
